# EUROPÄISCHE PATENTSCHRIFT

(11) **EP 1 121 077 B1**
(45) Veröffentlichungstag und Bekanntmachung des Hinweises auf die Patenterteilung: **22.10.2003**
(21) Anmeldenummer: 00954354.7
(22) Anmeldetag: 22.07.2000
(51) Int. Cl.: A61F 5/01

(54) **ORTHESENGELENK**
ORTHESIS JOINT
ARTICULATION D'ORTHESE

(30) Priorität: 17.08.1999 DE 29914375 U
(43) Veröffentlichungstag der Anmeldung: 08.08.2001
(60) Teilanmeldung: 03009209.2
(73) Patentinhaber: Medi Bayreuth Weihermüller & Voigtmann GmbH & Co. KG, 95448 Nürnberg (DE)
(72) Erfinder: WEIHERMÜLLER, Michael, D-95448 Bayreuth (DE)
(74) Vertreter: Schuhmann, Albrecht
(86) Internationale Anmeldenummer: DE0002385
(87) Internationale Veröffentlichungsnummer: WO01012110

(56) Entgegenhaltungen:
- EP-A- 0 693 276
- EP-A- 0 841 044
- US-A- 5 031 606
- US-A- 5 036 837

## Beschreibung

Die vorliegende Erfindung betrifft ein Orthesengelenk mit den Merkmalen des Oberbegriffs des Anspruchs 1.

Ein einstellbares Orthesengelenk ist beispielsweise aus der EP-A-0 841 044 bekannt Hier wird ein bestimmter voreinstellbarer Schwenkbereich der Orthese von einer Federkraft beaufschlagt, wobei die Feder für eine beaufschlagungsfreie Extensions- oder Flexionsbewegung außer Kraft gesetzt werden kann. Ein Orthesengelenk für Orthesen für zwei gegeneinander beug- und streckbare Körperteile, wie Knieorthesen oder Armorthesen, wobei das Orthesengelenk zwei Schienen drehbar miteinander verbindet, die jeweils mit oberen und unteren Orthesenteilen verbunden sind, die an dem jeweiligen Körperteil befestigbar sind und wobei an der Orthese jeweils lateral und/oder medial ein Schienenpaar mit Orthesengelenk angeordnet ist, wobei die Schienen endseitig über einen oder mehrere gemeinsame Zapfen oder durch endseitige Verzahnungen im Orthesengelenk miteinander in Eingriff stehen, und wobei für Streckung und/oder Beugung einstellbare Endanschläge vorhanden sind ist aus der EP-A-693 276 bekannt. Die EP-A-633 007 zeigt einen ähnlichen Aufbau, jedoch mit einem Klinkensystem.

Aufgabe der vorliegenden Erfindung ist es, ein Orthesengelenk zu schaffen, dessen Extensions- oder Flexionsbereich einstellbar ist, wobei die Bewegung in einem bestimmten Bereich beaufschlagungsfrei erfolgt, jedoch vor Erreichen des jeweiligen Endpunkts auf eine entgegen wirkende, bremsende Kraft stößt. Damit soll eine schlagartige Belastung der Bänder, insbesondere von Kreuzbändern, vermieden werden, indem der Patient vor Errreichen des Endpunkts auf spürbaren Widerstand stößt. Zugleich hat dies den Effekt, daß die Rückkehr in eine neutrale Position angeregt wird.

Diese Aufgabe wird mit den Merkmalen des kennzeichnenden Teils des Anspruchs 1 gelöst. Fortbildungen und vorteilhafte Ausführungen der Erfindung sind in den weiteren Ansprüchen umfaßt.

Die Bremsung erfolgt durch ein elastisches Element, wobei ein Mitnehmer ab Erreichen des bestimmten Winkelbereichs mit dem elastischen Element in Wirkverbindung kommt. Die Bremsung kann mechanisch durch Reibwirkung wenigstens zweier Teile erfolgen, die ab Erreichen des bestimmten Winkelbereichs in Eingriff miteinander kommen, wobei beispielsweise mit Reibbelägen beschichtete Platten verwendet werden können, deren Anlagedruck einstellbar gemacht werden kann. Es können auch reibende und elastische Elemente zusammenwirken, wie beispielsweise ein mit Federkraft .beaufschlagter Keil, wobei die Federkraft einstellbar sein kann. Das elastische Element kann eine Feder mit konstanter oder dynamischer Kennlinie sein oder aus einem natürlichen oder synthetischen Elastomer bestehen. Die Rückbewegung in die Ausgangsposition, d.h. im wesentlichen die Geradestellung des Gelenks, erfolgt ohne Beaufschlagung durch eine Bremskraft, die nur in Extensions- oder Flexionsrichtung wirkt.

Nach einer bevorzugten Ausführung der Erfindung besteht das Orthesengelenk in ans sich bekannter Weise aus den Endstücken zweier Schienen, die an den jeweiligen Orthesenteilen befestigt sind und die endseitig Verzahnungen aufweisen, die miteinander kämmen, wobei sich die Endstücke der Schienen jeweils auf Zapfen einer diese verbindenden Grundplatte drehen. Erfindungsgemäß ist wenigstens ein Zapfen drehfest mit wenigstens einem Mitnehmer verbunden, der ab Erreichen des bestimmten Winkelbereichs unmittelbar oder über ein Zwischenglied auf ein oder mehrere elastische Elemente wirkt Der Mitnehmer kann zum Beispiel ein Haken sein, der mit dem Ende einer Spiralfeder verbunden ist, die in einem Gehäuse auf der Grundplatte gelagert ist. Nach der bevorzugten Ausführung der Erfindung sind die Endstücke drehfest mit den Zapfen verbunden wobei der Mitnehmer eine exzentrische Scheibe ist, die drehfest mit den Zapfen verbunden ist und nach einem voreingestellten Weg mit einem Zwischenglied in reibenden Eingriff kommt, wobei schließlich die Scheibe eine in ihrer Drehrichtung erfolgende Bewegung des Zwischenglieds in Richtung einer auf dieses wirkenden Federkraft hervorruft. Das Zwischenglied wirkt auf eine Druckfeder, eine Blattfeder oder ein elastomeres Element, wobei die Kraft der Einwirkung des Zwischenglieds auf die Feder oder das elastomere Element einstellbar ist. Die Einstellung erfolgt durch eine Stellschraube, mit der zwischen dem Zwischenglied und dem elastischen Element eine gewünschte Vorspannung hergestellt wird. Die Einstellung der Endanschläge über Bohrungen oder Langlöcher der Endstücke erfolgt, in die Zapfen eingreifen, die ortsfest an einer Abdeckplatte für die Endstücke angeordnet sind.

Im folgenden wird die Erfindung anhand von Zeichnungen beispielhaft näher beschrieben. Es zeigen:
Fig. 1 schematisch die Anordnung einer Orthese im Kniebereich;
Fig. 2 einen Längsschnitt durch das Orthesengelenk;
Fig. 3 eine Draufsicht auf die Mitnahme- und Bremsmechanik des Orthesengelenks;
Fig. 4 eine Draufsicht auf das Orthesengelenk unter Weglassung der oberen Abdeckplatte.

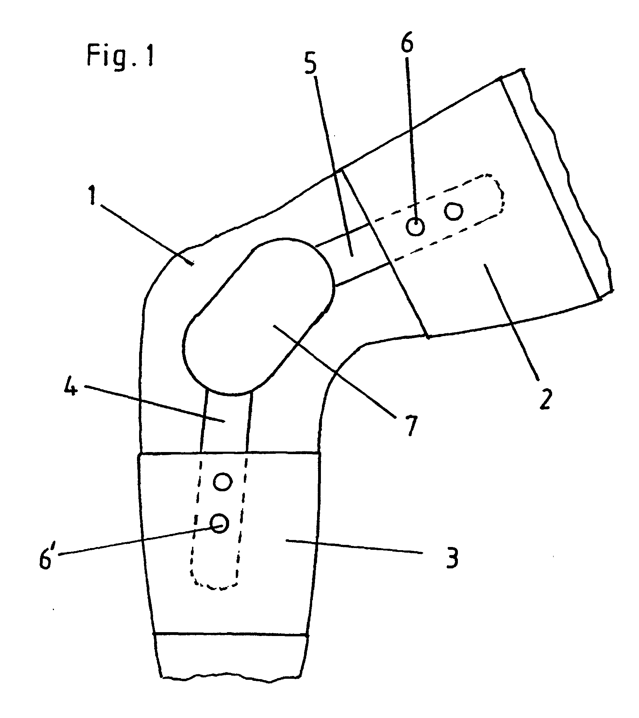

Fig. 1 zeigt ein Knie 1 mit einer angelegten Knieorthese. Die Knieorthese besteht aus einem Halteteil 2 für den Oberschenkel und einem Halteteil 3 für den Unterschenkel. In den Halteteilen sind lateral und medial jeweils Metallschienen 4, 5 mittels Nieten 6 befestigt, wobei sich die Schiene 5 des Halteteils 2 für den Oberschenkel nach unten und die Schiene 4 des Halteteils 3 für den Unterschenkel nach oben erstrecken. Beide Schienen vereinigen sich in dem Orthesengelenk 7, in dem sie über einen bestimmten Winkelbereich drehbar gelagert sind und über einc Verzahnung miteinander in Eingriff stehen.

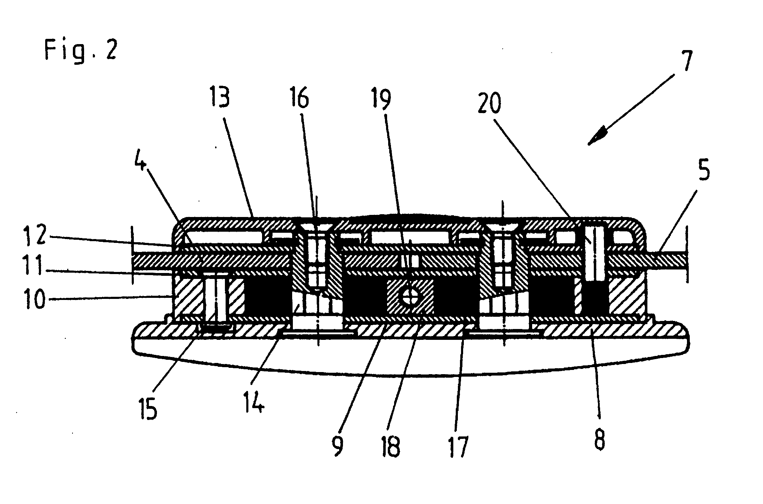

Fig. 2 zeigt den Aufbau des Orthesengelenks 7 im Längsschnitt. Es besteht aus einer Grundplatte 8, in der zwei nach oben gerichtete Sechskantzapfen 14 drehbar gelagert sind. Auf derGrundplatte liegt eine erste Zwischenplatte 9, an die sich ein flaches Gehäuse 10 anschließt. Das Gehäuse 10 beherbergt die Mitnahme- und Bremsmechanik, die weiter unten näher beschrieben wird. Das Gehäuse wird von einer zweiten Zwischenplatte 12 abgedeckt, durch die die Sechskantzapfen 14 drehbar hindurch geführt sind. Auf der zweiten Zwischenplatte 11 und unter einer dritten Zwischenplatte 12 legen die Endstücke der Schienen 4, 5, die über Verzahnungen miteinander in Eingriff stehen. Die Endstücke der Schienen 4, 5 sind drehfest mit den Sechskantzapfen 14 verbunden, die über den Schienen Schultern aufweisen, mit denen sie unter der dritten Zwischenplatte 12 anliegen, jedoch mit einem verjüngten zylindrischen Ende drehbar durch diese hindurch geführt sind. Auf der dritten Zwischenplatte 12 ist eine Abdeckplatte 13 angeordnet, die durch Schrauben 16 mit den Sechskantzapfen 14 verbunden ist, wobei sich die Schrauben 16 in der Abdeckplatte 13 drehen können. Die Grundplatte 8 ist über Schrauben oder Stifte 15 mit den unteren beiden Zwischenplatte 9, 11 und dem dazwischen liegenden Gehäuse 10 verbunden, so daß das Orthesengelenk 7 eine von oben und unten gesicherte Einheit darstellt.

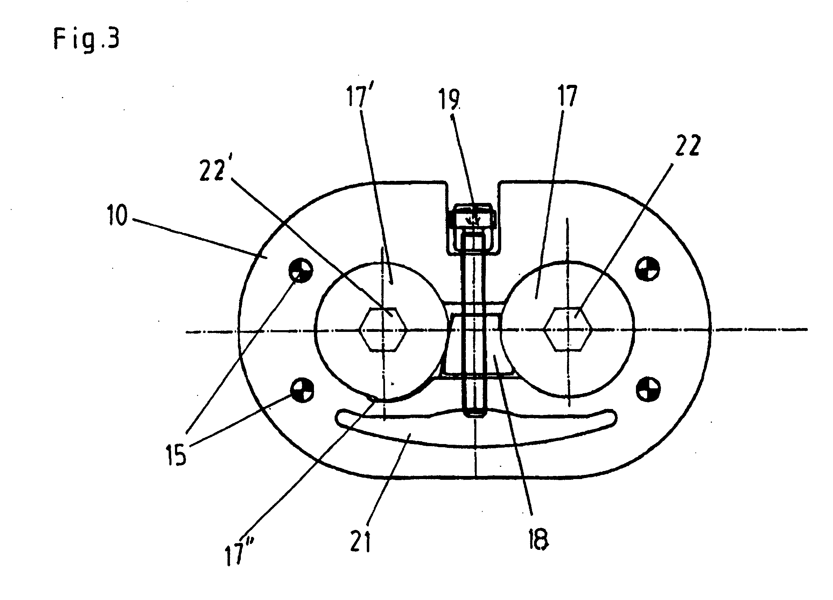

Fig. 3 zeigt eine Draufsicht auf das Gehäuse 10 mit seinen Mechanikteilen, wobei alle anderen Elemente des Orthesengelenks weggelassen sind. Zu erkennen sind vier untere Befestigungsstifte 15, die seitlich an den Enden angeordnet sind. Es sind zwei Ausnehmungen vorhanden, in denen in einem Abstand zueinander in der Längsachse des Gelenks zwei Scheiben 17, 17' angeordnet sind. Die Scheiben weisen zentrale Sechskantbohrungen 22,22' auf, mit denen sie drehfest auf die (hier nicht gezeigten) Sechskantzapfen gesteckt sind. Solche Sechskantbohrungen weisen auch die (hier nicht gezeigten) Endstücke der Schienen auf, so daß sich, wenn sich die Schiene zueinander verdrehen, die Scheiben 17, 17' zwangsläufig mitdrehen. Die rechte Scheibe 17 ist kreisrund ausgeführt, während die linke Scheibe einen exzentrischen Abschnitt aufweist. Zwischen den Scheiben ist ein Keil 18 angeordnet, der mit einer Gewindebohrung verdrehsicher zwischen den (hier nicht gezeigten) Zwischenplatten auf einem Gewindebolzen läuft. Der Gewindebolzen 19 ist mit seinem inneren Ende an einem elastischen Element 21 gelagert, das ihn mit Federkraft beaufschlagt und nach außen zu drücken trachtet. Dabei nimmt der Gewindebolzen 19 den Keil 18 mit, der mit seinen Keilflächen als Bremskeil an der Außenkontur der Scheiben 17, 17' anliegt. Je tiefer der Gewindebolzen 19 eingeschraubt wird, desto höher ist die auf ihn und den Keil 18 wirkende Kraft. Während zwischen der kreisrunden Scheibe 17 und dem Keil 18 eine im wesentlichen gleichbleibende Reibung vorhanden wäre, kommt bei einer Drehung der linken Scheibe 17' entgegen dem Uhrzeigersinn deren sich exzentrisch vergrößernder Außenumfang in eine immer größer werdende Reibung mit dem Keil 18, die gleichzeitig auf die kreisrunde Scheibe 17 übertragen wird, so daß sich zwischen dem Keil und den Scheiben eine stärker werdende Bremskraft bis zur Blockade einstellen würde, würde der Weg der Scheiben nicht durch die unten geschilderten Maßnahmen begrenzt. Durch diese Begrenzung kommt es nach einem Bereich geringer Reibung nach Eingriff eines Teils des exzentrischen Bereichs der linken Scheibe zu einem Bereich größerer Reibung, was sich für den Benutzer so darstellt, daß er bei beginnender Flexion oder Extension zunächst keinen oder nur geringen Widerstand spürt, der vor Erreichen der Endstellung durch ein spürbares Abbremsen abgelöst wird. Die Form des Keils 18 bedingt, daß dieser nur in einer Richtung bremsend wirkt, in der er auf die Scheiben gepreßt wird. In der anderen Richtung, d.h. bei einer Bewegung zurück, übt der Keil keine Bremskraft aus.

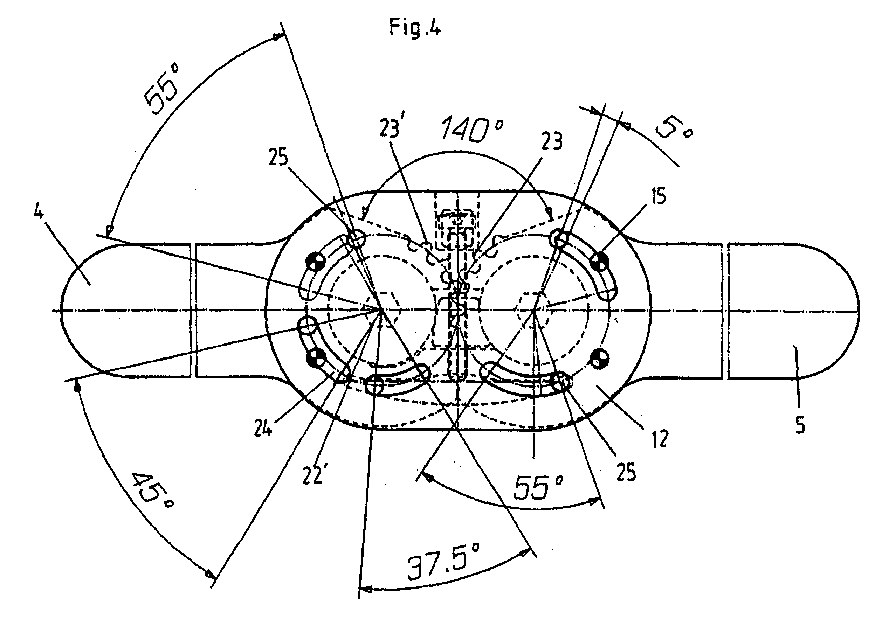

Fig. 4 zeigt die Endstücke der Schienen 4,5 mit ihren Verzahnungen 23, 23', die miteinander kämmen. Beide Endstücke weisen Sechskantbohrungen 22' auf, mit denen sie, wie geschildert, drehfest mit den (hier nicht gezeigten) Sechskantzapfen verbunden sind. Dadurch sind sie mit der Mitnahme- und Bremsmechanik verbunden, die zu Fig. 3 beschrieben wurde. Auf den Endstücken liegt die dritte Zwischenplatte 12, die eine Anzahl von Bohrungen 25 aufweist, sowie die Enden der unteren Befestigungsstifte 15. Die hier dargestellten Langlöcher 24, die konzentrisch zu den Drehachsen der Enden der Schienen ausgerichtet sind, sind nicht in der Zwischenplatte 12, sondern in den Endstücken der Schienen angeordnet, was nur zur Verdeutlichung zeichnerisch unkorrekt dargestellt ist, um ihr Zusammenwirken mit den Bohrungen 25 zu verdeutlichen. Die Drehachsen der Schienen 4, 5 liegen im wesentlichen in der Längsachse der Sechskantbohrungen 22'. Zur Einstellungen von Endanschlägen für die Schienen 4, 5 können Stifte (Stifte 20 in Fig. 2) durch die Bohrungen 25 gesteckt werden, die in der oberen Abdeckung gehalten werden. Diese Stifte laufen dann in gewünschten Langlöchern, so daß die Wahl von zulässigen Beuge- und Streckwinkeln für das Knie möglich ist. Bestimmte, beispielhafte Winkelbereich sind hier dargestellt. Da die Anordnung von Bohrungen und Langlöchern wie gewünscht ausgeführt werden kann, sind selbstverständlich andere Winkelbereiche möglich. Durch das Zusammenwirken der Mitnahme- und Bremsmechanik mit den Endanschlägen stellt sich der oben genannte Effekt für den Benutzer ein.

## Patentansprüche

1. Orthese mit Orthesengelenk für zwei gegeneinander beug- und streckbare Körperteile, wie Knieorthesen oder Armorthesen,
wobei das Orthesengelenk (7) zwei Schienen (4,5) drehbar miteinander verbindet, die jeweils mit oberen und unteren Orthesenteilen (2,3) verbunden sind, die an dem jeweiligen Körperteil befestigbar sind und wobei an der Orthese jeweils lateral und/oder medial ein Schienenpaar mit Orthesengelenk angeordnet ist,
wobei die Schienen endseitig über einen oder mehrere gemeinsame Zapfen oder durch endseitige Verzahnungen (23,23') im Orthesengelenk (7) miteinander in Eingriff stehen, wobei für Streckung und/oder Beugung einstellbare Endanschläge (20) vorhanden sind, und wobei die Endanschläge (20) über einen Winkelbereich verstellbar sind,
**dadurch gekennzeichnet,**
**daß** die Beugung, bzw. Streckung in dem Orthesengelenk (7) in einem bestimmten Winkelbereich beaufschlagungsfrei erfolgt und in einem auschließenden Winkelbereich α eine Bremsung erfährt, wobei α einen Winkel von zwischen 3° und 25° darstellt,
und **daß** die Bremsung durch ein elastisches Glied (21) erfolgt, wobei ein Mitnehmer (17') ab Erreichen des bestimmten Winkelbereichs mit dem elastischen Glied (21) in Wirkverbindung kommt, und mit zunehmendem Winkelbereich die Bremskraft zwischen dem elastischen Glied (21) und dem Mitnehmer stärker wird.

2. Orthese nach Anspruch 1,
**dadurch gekennzeichnet,**
**daß** die Bremsung mechanisch durch Reibwirkung wenigstens zweier Teile erfolgt, die ab Erreichen des bestimmten Winkelbereichs in Eingriff miteinander kommen.

3. Orthese nach Anspruch 1,
**dadurch gekennzeichnet, daß** das elastische Element eine Feder mit konstanter oder dynamischer Kennlinie ist.

4. Orthese nach Anspruch 1,
**dadurch gekennzeichnet,**
**daß** das elastische Element aus einem Elastomer besteht.

5. Orthese nach einem der vorhergehenden Ansprüche,
**dadurch gekennzeichnet,**
**daß** die Bewegung in die Ausgangsposition ohne Beaufschlagung durch eine Bremskraft erfolgt.

6. Orthese nach einem der vorhergehenden Ansprüche,
**dadurch gekennzeichnet,**
**daß** die Endstücke der Schienen (4, 5) sich jeweils auf Zapfen (14) einer diese verbindenden Grundplatte (8) drehen.

7. Orthese nach Anspruch 6,
**dadurch gekennzeichnet,**
**daß** wenigstens ein Zapfen (14) drehfest mit wenigstens einem Mitnehmer (17, 17') verbunden ist, der ab Erreichen des bestimmten Winkelbereichs unmittelbar oder über ein Zwischenglied (18) auf ein oder mehrere elastische Elemente (21) wirkt.

8. Orthese nach Anspruch 7,
**dadurch gekennzeichnet,**
**daß** der Mitnehmer ein Haken ist, der mit dem Ende einer Spiralfeder verbunden ist,
und **daß** die Spiralfeder in einem Gehäuse auf der Grundplatte gelagert ist.

9. Orthese nach Anspruch 7,
**dadurch gekennzeichnet,**
**daß** die Endstücke drehfest mit den Zapfen (14) verbunden sind,
**daß** der Mitnehmer eine exzentrische Scheibe (17') ist, die drehfest mit den Zapfen (14) verbunden ist,
**daß** die Scheibe (17') nach einem voreingestellten Weg mit einem Zwischenglied (18) in reibenden Eingriff kommt,
und **daß** die Scheibe (17') eine in ihrer Drehrichtung erfolgende Bewegung des Zwischenglieds (18) in Richtung einer auf dieses wirkenden Federkraft hervorruft.

10. Orthese nach Anspruch 9,
**dadurch gekennzeichnet,**
**daß** das Zwischenglied (18) auf eine Druckfeder, eine Blattfeder oder ein elastomeres Element wirkt.

11. Orthese nach Anspruch 10,
**dadurch gekennzeichnet,**
**daß** die Kraft der Einwirkung des Zwischenglieds (18) auf die Feder oder das elastomere Element einstellbar ist.

12. Orthese nach Anspruch 11,
**dadurch gekennzeichnet,**
**daß** die Einstellung durch eine Stellschraube (19) erfolgt, mit der zwischen dem Zwischenglied und dem elastischen Element eine gewünschte Vorspannung hergestellt wird.

13. Orthese nach Anspruch 9,
**dadurch gekennzeichnet,**
**daß** die Einstellung der Endanschläge über Bohrungen oder Langlöcher (24) der Endstücke erfolgt, in die Zapfen (20) eingreifen, die ortsfest an einer Abdeckplatte für die Endstücke angeordnet sind.

## Claims

1. An orthosis comprising an orthosis hinge for two body parts which are able to flex and extend in relation to one another, such as knee orthoses or arm orthoses,
wherein the orthosis hinge (7) rotatably joins together two bars (4, 5) joined respectively to upper and lower orthosis parts (2, 3), the said parts being securable on the relevant body part, and wherein a pair of bars with the orthosis hinge is arranged on the orthosis each laterally and/or medially thereof,
wherein the end faces of the bars are in engagement with one another in the orthosis hinge (7) by way of one or more common pivots or by means of teeth (23, 23') on the end face thereof,
wherein there are adjustable end stops (20) for extension and/or flexion, and wherein the end stops (20) are movable over an angular range,
**characterised in that** the flexion or extension in the orthosis hinge (7) is effected free of action thereon in a specific angular range, and in a subsequent angular range α experiences braking, α representing an angle of between 3° and 25°,
and **in that** the braking is effected by means of a resilient element (21), wherein a driver (17') comes into active connection with the resilient element (21) from when the specific angular range is reached, and as the angular range increases, the braking force between the resilient element (21) and the driver becomes stronger.

2. An orthosis according to Claim 1,
**characterised in that** the braking is effected mechanically as a result of the frictional effect of at least two parts which come into engagement with one another from when the specific angular range is reached.

3. An orthosis according to Claim 1,
**characterised in that** the resilient element is a spring with a constant or dynamic characteristic.

4. An orthosis according to Claim 1,
**characterised in that** the resilient element is made of an elastomer.

5. An orthosis according to any one of the preceding Claims,
**characterised in that** the movement into the starting position is effected without action thereon by a braking force.

6. An orthosis according to any one of the preceding Claims,
**characterised in that** the end sections of the bars (4, 5) each turn on pivots (14) of a base plate (8) connecting the said pivots.

7. An orthosis according to Claim 6,
**characterised in that** at least one pivot (14) is joined, fixed rotationally, to at least one driver (17, 17'), which acts on one or more resilient elements (21) directly or by way of an intermediary element (18) from when the specific angular range is reached.

8. An orthosis according to Claim 7,
**characterised in that** the driver is a hook which is joined to the end of a helical spring,
and **in that** the helical spring is mounted in a housing on the base plate.

9. An orthosis according to Claim 7,
**characterised in that** the end sections are joined, fixed rotationally, to the pivots (14),
**in that** the driver is an eccentric disc (17') which is joined, fixed rotationally, to the pivots (14),
**in that** the disc (17') comes into frictional engagement with an intermediary element (18) after a preset distance,
and **in that** the disc (17') causes a movement of the intermediary element (18), occurring in the rotational direction of the said disc, in the direction of a spring force acting on the said element.

10. An orthosis according to Claim 9,
**characterised in that** the intermediary element (18) acts on a compression spring, a leaf spring or an elastomeric element.

11. An orthosis according to Claim 10,
**characterised in that** the force of the action of the intermediary element (18) on the spring or the elastomeric element is adjustable.

12. An orthosis according to Claim 11,
**characterised in that** the adjustment is effected by means of an adjusting screw (19) with which a desired initial stress is created between the intermediary element and the resilient element.

13. An orthosis according to Claim 9,
**characterised in that** the adjustment of the end stops is effected via bores or longitudinal slots (24) of the end sections, into which pins (20) engage, the said pins being arranged so as to be fixed in position on a cover plate for the end sections.

## Revendications

1. Orthèse avec articulation d'orthèse, destinée à deux parties du corps aptes à s'étendre et à fléchir l'une par rapport à l'autre, telle que des orthèses de genou ou des orthèses de bras, dans laquelle
l'articulation d'orthèse (7) relie deux attelles (4, 5) de façon à pourvoir tourner, lesquelles sont reliées respectivement à la pièce d'orthèse supérieure et à la pièce d'orthèse inférieure (2, 3) qui peuvent être fixées à la partie du corps correspondante, et une paire d'attelles dotée de l'articulation d'orthèse est disposée sur l'orthèse en position latérale et/ou médiane,
les attelles sont en engagement l'une avec l'autre du côté de l'extrémité par une ou plusieurs chevilles communes ou bien par des endentements (23, 23') situés du côté de l'extrémité dans l'articulation d'orthèse (7),
des butées d'extrémité (20) étaient réglables en vue de l'extension et/ou de la flexion, et
les butées d'extrémité (20) sont réglables sur une plage angulaire,
**caractérisé en ce que**
la flexion resp. l'extension dans l'articulation d'orthèse (7) est effectuée sans contrainte dans une plage angulaire déterminée et subit dans une plage angulaire α un freinage, α étant un angle compris entre 3° et 25°, et **en ce que**
le freinage est effectué par un élément élastique (21), un dispositif d'accouplement (17') venant en liaison active avec l'élément élastique (21) à partir du moment où la plage angulaire déterminée est atteinte, et à mesure que la plage angulaire augmente, la force de freinage entre l'élément élastique (21) et le dispositif d'accouplement devient plus importante.

2. Orthèse selon la revendication 1, **caractérisée en ce que** le freinage est effectué mécaniquement par friction d'au moins deux pièces qui viennent en engagement l'une avec l'autre à partir du moment où la plage angulaire déterminée est atteinte.

3. Orthèse selon la revendication 1, **caractérisée en ce que** l'élément élastique est un ressort présentant une caractéristique constante ou dynamique.

4. Orthèse selon la revendication 1, **caractérisée en ce que** l'élément élastique se compose d'un élastomère.

5. Orthèse selon l'une des revendications précédentes, **caractérisée en ce que** le mouvement dans la position de départ est effectué sans contrainte exercée par une force de freinage.

6. Orthèse selon l'une des revendications précédentes, **caractérisée en ce que** les pièces d'extrémité des attelles (4, 5) tournent chacune sur des chevilles (14) d'une plaque de base (8) qui relie lesdites attelles.

7. Orthèse selon la revendication 7, **caractérisée en ce qu'**au moins une cheville (14) est reliée fixe en rotation à au moins un dispositif d'accouplement (17, 17') qui agit directement ou par un élément intermédiaire (18) sur un ou plusieurs éléments élastiques (21).

8. Orthèse selon la revendication 7, **caractérisée en ce que** le dispositif d'accouplement est un crochet qui est relié à l'extrémité d'un ressort en spirale, et
**en ce que** le ressort en spirale est logé dans un boîtier sur la plaque de base.

9. Orthèse selon la revendication 7, **caractérisée en ce que** les pièces d'extrémité sont reliées fixes en rotation aux chevilles (14),
**en ce que** le dispositif d'accouplement est un disque excentrique (17') qui est relié fixe en rotation à la cheville (14),
**en ce que** le disque (17') est en engagement par friction avec un élément intermédiaire (18) suivant un trajet préréglé, et
**en ce que** le disque (17') provoque un mouvement de rotation de l'élément intermédiaire (18) en direction d'une force de ressort agissant sur ledit élément intermédiaire (18).

10. Orthèse selon la revendication 9, **caractérisée en ce que** l'élément intermédiaire (18) agit sur un ressort de compression, un ressort à lame ou bien un élément élastomère.

11. Orthèse selon la revendication 10, **caractérisée en ce que** la force d'action de l'élément intermédiaire (18) sur le ressort où l'élément élastomère est réglable.

12. Orthèse selon la revendication 11, **caractérisée en ce que** le réglage est effectué par une vis de réglage (19) permettant de réaliser une précontrainte souhaitée entre l'élément intermédiaire et l'élément élastique.

13. Orthèse selon la revendication 9, **caractérisée en ce que** le réglage des butées d'extrémité est effectué par des perçages ou des trous oblongs (24) des pièces de butée dans lesquels s'engagent des chevilles (20) qui sont disposées fixes sur une plaque de recouvrement destinée aux pièces de butée.
